(19) Europäisches Patentamt
European Patent Office
Office européen des brevets

(11) Veröffentlichungsnummer: **0 205 131**
**B1**

# (12) EUROPÄISCHE PATENTSCHRIFT

(45) Veröffentlichungstag der Patentschrift: **21.09.88**

(51) Int. Cl.⁴: **C 07 C 121/43**, C 07 C 120/00

(21) Anmeldenummer: **86107782.4**

(22) Anmeldetag: **07.06.86**

## (54) Verfahren zur Herstellung von 2-(N-Formylamino)-propionitril.

(30) Priorität: **12.06.85 DE 3520982**

(43) Veröffentlichungstag der Anmeldung: **17.12.86 Patentblatt 86/51**

(45) Bekanntmachung des Hinweises auf die Patenterteilung: **21.09.88 Patentblatt 88/38**

(84) Benannte Vertragsstaaten: **BE CH DE FR GB IT LI NL**

(56) Entgegenhaltungen:
**DD-A-214 125**
**DE-B-1 963 010**
**DE-B-2 226 588**
**DE-C-734 725**
**DE-C-735 771**
**US-A-2 461 842**

(73) Patentinhaber: **BASF Aktiengesellschaft, Carl-Bosch- Strasse 38, D-6700 Ludwigshafen (DE)**

(72) Erfinder: **Bewert, Wolfgang, Dr., Lorscher Ring 8 c, D-6710 Frankenthal (DE)**
Erfinder: **Kiefer, Hans, Dr., Im Sandgarten 5, D-6706 Wachenheim (DE)**

**EP 0 205 131 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein verbessertes Verfahren zur Herstellung vor 2-(N-Formylamino)-propionitril durch Umsetzung von Acrylnitril mit Formamid in Gegenwart einer basischen Verbindung:

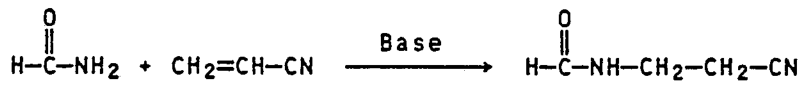


Aus der DE-PS-734 725 ist es bekannt, bei dieser Reaktion Alkalimetallbasen wie Natriumhydroxyd und Natrium als basische Katalysatoren zu verwenden, jedoch sind die hierbei erzielbaren Ausbeuten von 48 - 55 % unbefriedigend wie eigene Versuche ergeben haben Nach dem in Bull. Soc. Chim. France 1957, Seite 1108 beschriebenen Verfahren nimmt man die Umsetzung in Gegenwart basischer quartäre Ammoniumgruppen enthaltender Ionenaustauscher vor, erhält hierbei allerdings größere Mengen an Bis-(2-N-Formylimino)-propionitril als unerwünschtes Nebenprodukt.

Es ist ferner bekannt, 2-Aminopropionitril durch die sog. Cyanethylierung von Ammoniak herzustellen (Houben-Weyl, "Methoden der Organischen Chemie", Band XI/1, S. 272 und Band XI/2, S. 496), so daß man auf diesem Wege durch Formylierung von dieser Verbindung auch zu der Titelverbindung gelagen könnte, jedoch hat diese Methode den gravierenden Nachteil, daß man hierbei stets auch die Bis- und Triscyanethylamine als Nebenprodukte erhält.

Der Erfindung lag daher die Aufgabe zugrunde, das 2-(N-Formylamino)-propionitril auf wirtschaftlichere Weise zugänglich zu machen als bisher.

Demgemäß wurde ein verbessertes Verfahren zur Herstellung von 2-(N-Formylamino)-propionitril durch Umsetzung von Acrylnitril mit Formamid in Gegenwart einer basischen Verbindung gefunden, welches dadurch gekennzeichnet ist, daß man hierbei als basische Verbindung eine tertiäre Stickstoffbase verwendet.

Als tertiäre Stickstoffbasen eignen sich besonders solche mit einem $p_K$-Wert der kleiner als 5 ist, also beispielsweise

- aliphatische tertiäre Amine wie Trimethylamin ($p_K = 4.26$), Triethylamin ($p_K = 3.13$), Tripropylamin ($p_K = 3.35$). Tributylamin ($p_K = 3.11$), Dimethyldodecylamin und N,N,N', N'-Tetramethyl-1,3-diaminopropan
- cycloaliphatisch-aliphatische tertiäre Amine wie N-Methylpiperidin und N,N'-Dimethylpiperazin und
- bicyclische tertiäre Amine 1,4-Diazabicyclo-[2.2.2]-octan ("DABCO" $p_K = 2.95$).

Die Menge der katalytisch wirkenden Stickstoffbasen ist nicht kritisch, da sie lediglich einen Einfluß auf die Reaktionsgeschwindigkeit hat. Im allgemeinen liegt diese Menge zur Erzielung eines hinreichend schnellen Umsatzes zwischen 1 und 50 mol-% pro Mol Acrylnitril.

Formamid und Acrylnitril reagieren stöchiometrisch miteinander, jedoch ist es zweckmäßig, das Formamid in einem bis zu etwa 20-fachen molaren Überschuß über das Acrylnitril einzusetzen, wobei das bevorzugte Molverhältnis zwischen 2 : 1 und 10 : 1 liegt.

Um die Polymerisation des Acrylnitrils nach Möglichkeit zu unterdrücken, empfiehlt sich die Mitverwendung eines Polymerisationsinhibitors wie Hydrochinon, Hydrochinonmonomethylether, 3-tert.-Butyl-4-hydroxyanisol, 2-6-Di-tert.-butyl-4-methyl-phenol oder eines Gallussäureesters in Konzentrationen von etwa 0,001 - 0,5 Gew.-% des Acrylnitrils.

Die Mitverwendung eines Lösungsmittels ist im allgemeinen nicht erforderlich, kann aber insbesondere dann von Vorteil sein, wenn sich die Reaktionspartner nicht ausreichend ineinander lösen.

Als Lösungsmittel eignen sich beispielsweise polare Lösungsmittel wie Dimethylformamid, Dimethylacetamid, N-Methylpyrrolidon, Tetrabutylharnstoff, Dioxan und Tetrahydrofuran.

Aus verfahrenstechnischen Gründen wird das Arbeiten bei Normaldruck bevorzugt, jedoch kann man auch unter leicht erhöhtem Druck - etwa bis zu 5 bar - arbeiten, z. B. wenn man ein sehr leicht flüchtigen tertiäres Amin oder ein leichtflüchtiges Lösungsmittel verwendet.

Führt man die Umsetzung diskontinuierlich aus, legt man das Formamid, die Stickstoffbase und gegebenenfalls das Lösungsmittel zweckmäßigerweise vor und gibt hierzu allmählich das Acrylnitril oder eine Lösung des Acrylnitrils. Ähnlich verfährt man bei kontinuierlicher Betriebsweise, wobei man Formamid und die Stickstoffbase einerseits und das Acrylnitril andererseits in etwa konstantem Molverhältnis zusammenführt.

Im übrigen bietet das erfindungsgemäße Verfahren keine verfahrenstechnischen Besonderheiten, so daß nähere Ausführungen hierüber entbehrlich sind. Das gleiche gilt für die Aufarbeitung des Reaktionsgemisches.

Das 2-(N-Formylamino)-propionitril ist bekanntermaßen ein wichtiges Vorprodukt für die Synthese von Vitamin $B_1$ und außerdem eröffnet es einen vorteilhaften Zugang zu dem ebenso wichtigen unmittelbaren Folgeprodukt β-Alanin.

**Beispiele 1 bis 10**

Es wurde jeweils eine Lösung aus 450 g (10 mol) Formamid und a g (a' mol) einer tertiären Stickstoffbase vorgelegt und bei $T_1$°C im Laufe von etwa 1 Stunde mit 53 g (1 mol) Acrylnitril versetzt, welches mit 40 ppm Hydrochinon-monomethylether stabilisiert war. Anschließend wurde das Reaktionsgemisch noch weitere 3 Stunden unter Rückflußkühlung erhitzt, wobei die Temperatur auf $T_2$°C anstieg oder auf $T_1$ gehalten wurde ($T_2 = T_1$).

Die übliche destillative Aufarbeitung lieferte das 2-(N-Formylamino)-propionitril in einer Ausbeute von y % (Sdp. 130°C/1 mbar), wobei die nicht umgesetzten Einsatzstoffe praktisch quantitativ zurückgewonnen wurden. Der Anteil der Nebenprodukte lag zwischen etwa 0,5 und 3 %, bezogen auf das eingesetzte Acrylnitril.

Einzelheiten dieser Versuche sowie deren Ergebnisse sind der nachstehenden Tabelle zu entnehmen.

| Bsp. Nr. | tert. Stickstoffbase | a [g] | a' [mol] | $T_1$ [°C] | $T_2$ [°C] | y [%] |
|---|---|---|---|---|---|---|
| 1 | Triethylamin | 20 | 0,2 | 80 | 100 | 83 |
| 2 | Tri-n-propylamin | 20 | 0,14 | 130 | 130 | 81 |
| 3 | N,N,N',N'-Tetramethyl-1,3-diamion-propan | 3 | 0,02 | 130 | 130 | 76 |
| 4 | 1,4-Diazabicyclo-[2.2.2]-octan | 12 | 0,09 | 130 | 130 | 76 |
| 5 | Tri-n-butylamin | 20 | 0,11 | 130 | 130 | 75 |
| 6 | Dimethyl-isopropylamin | 10 | 0,12 | 115 | 130 | 75 |
| 7 | 1,8-Diazabicyclo-[5,4,0]-unde-7-en | 10,5 | 0,07 | 130 | 130 | 72 |
| 8 | N,N,N',N',N''-Pentamethyl-diethylen-triamin | 3 | 0,017 | 120 | 130 | 75 |
| 9 | N,N,N',N'-Tetramethyl-2,2'-diamino-diethylether | 20 | 0,13 | 120 | 130 | 73 |
| 10 | N-Methylpiperidin | 14 | 0,14 | 125 | 130 | 71 |

**Patentansprüche**

1. Verfahren zur Herstellung von 2-(N-Formylamino)-propionitril durch Umsetzung von Acrylnitril mit Formamid in Gegenwart einer basischen Verbindung, dadurch gekennzeichnet, daß man hierbei als basische Verbindung eine tertiäre Stickstoffbase verwendet.

2. Verfahren nach Anspruch 1, dadurch gekennzeichnet daß die tertiäre Stickstoffbase einen $p_K$-Wert hat, der kleiner als 5 ist.

**Claims**

A process for the preparation of 2-(N-formylamino)-propionitrile by reacting acrytonitrile with formamide in the presence of a basic compound, wherein the basic compound used is a tertiary nitrogen base.

2. A process as claimed in claim 1, wherein the tertiary nitrogen base has a $p_K$ which is less than 5.

**Revendications**

1. Procede de préparation de 2-(N-formylamino)-propionitrile par réaction d'acrylonitrile avec le formamide en présence d'un composé basique, caracterisé en ce qu'on y utilise, comme composé basique, une base azotée tertiaire.

2. Procédé selon la revendication 1, caractérisé en ce que la base azotée tertiaire a un $p_K$ qui est inférieur à 5.